# EUROPÄISCHE PATENTSCHRIFT

(11) **EP 1 533 034 B1**
(45) Veröffentlichungstag und Bekanntmachung des Hinweises auf die Patenterteilung: **23.01.2008**
(21) Anmeldenummer: 04023985.7
(22) Anmeldetag: 08.10.2004
(51) Int. Cl.: B01L 3/00

(54) **Vorrichtung zur Übertragung einer Referenzflüssigkeit in ein Messgerät, Messgerät mit einer solchen Vorrichtung und Verfahren zur Übertragung einer Referenzflüssigkeit in ein Messgerät**
Apparatus for transfering a reference liquid to a measuring instrument, measuring instrument with such apparatus and process of transfering a reference liquid to a measuring instrument
Dispositif de transférer d'un liquide de référence dans un instrument de mesure, instrument de mesure avec ce dispositif et procédé de transférer d'un liquide de référence dans un instrument de mesure

(30) Priorität: 18.11.2003 DE 10353937
(43) Veröffentlichungstag der Anmeldung: 25.05.2005
(73) Patentinhaber: Fresenius Medical Care Deutschland GmbH, 61352 Bad Homburg v.d.H. (DE)
(72) Erfinder: Mager, Gerhard, Dr., 61352 Bad Homburg (DE); Fischer, Michael, 08060 Zwickau (DE)
(74) Vertreter: Luderschmidt, Schüler & Partner

(56) Entgegenhaltungen:
- US-A- 4 275 774
- US-A- 4 361 253

## Beschreibung

Die vorliegende Erfindung betrifft eine Vorrichtung und ein Verfahren zum Einbringen einer Referenzflüssigkeit in ein Messgerät zur Ermittlung der Parameter von Flüssigkeitsproben. Ferner betrifft die Erfindung ein Messgerät mit der vorgenannten Vorrichtung.

Aus dem Stand der Technik sind unterschiedliche Messgeräte zur Ermittlung der Parameter von Flüssigkeitsproben bekannt, wie beispielsweise Messgeräte zur Ermittlung der Parameter bzw. Eigenschaften von Blutproben. So können beispielsweise Blutgase (pO2, pCO2, pH), Elektrolyte (Na, K, Ca, Cl) und die Leitfähigkeit einer Blutprobe mit Hilfe derartiger Messgeräte ermittelt werden.

Insbesondere in der Medizintechnik ist es erforderlich, dass die Messgeräte sehr genaue Messwerte von beispielsweise den physiologischen Blutparametern eines Patienten liefern. Aus diesem Grunde müssen die Messgeräte regelmäßig auf deren Messgenauigkeit hin überprüft werden. Zur Qualitätskontrolle wird das komplette Messsystem einschließlich seiner Kalibriermedien und Kalibriefunktionen auf korrekte Funktion überprüft.

Zur Überprüfung der Messgenauigkeit wird eine Probe mit bekanntem Gehalt aller Analyte vermessen. Unterschreiten die Abweichungen zwischen Messwert und vorgegebenem Wert einen bestimmten Betrag, so kann von einer korrekten Funktion des Messsystems ausgegangen werden. Sind die Abweichungen größer als zulässig, darf mit dem Messsystem keine Patientenprobe mehr gemessen werden, bis durch entsprechende Reparatur- oder Wartungsmaßnahmen wieder ein Zustand herbeigeführt worden ist, in dem die Kriterien der Qualitätskontrollmessung erfüllt werden. Die Qualitätskontrollmessung und ihre Ergebnisse müssen dokumentiert werden.

Die Referenzflüssigkeit zur Überprüfung der Messgenauigkeit wird im Allgemeinen in verschlossenen Ampullen bereitgestellt, die erst unmittelbar vor der Benutzung geöffnet werden, um Verunreinigungen oder sonstige Beeinflussungen der Referenzflüssigkeit zu vermeiden. Um die Messergebnisse an den Referenzflüssigkeiten nicht zu verfälschen, muss beim Eingeben der Referenzflüssigkeit in das Messgerät besonders sorgfältig vorgegangen werden. Im Stand der Technik kommen dabei vorwiegend die nachfolgend beschriebenen Verfahren zur Anwendung.

Um die Referenzflüssigkeit aus der Ampulle zu entnehmen, kommt häufig eine Spritze mit aufgesetzter Hohlnadel zum Einsatz. Die Hohlnadel wird dabei in die Ampulle eingeführt, um die Referenzflüssigkeit anschließend in den Spritzenzylinder zu saugen. Ist der Spritzenzylinder ausreichend gefiillt, so kann die Hohlnadel von der Spritze entfernt werden. Danach hält die Bedienungsperson die Spritze mit der Öffnungsseite nach oben und klopft mehrmals leicht gegen den Spritzenzylinder, damit sich die Luft, die zwangsläufig durch den Saugvorgang in die Referenzflüssigkeit gelangt, im vorderen Abschnitt des Spritzenzylinders sammelt. Abschließend wird der Spritzenkolben so weit nach vorne geschoben bis die im vorderen Abschnitt gesammelte Luft gänzlich herausgedrückt wurde. Eine derart vorbereitete Spritze kann nunmehr in einen Eingabeport des Messgerätes eingeführt werden, um die Referenzflüssigkeit anschließend einzuspritzen. Bei dem bekannten Verfahren kann auch auf eine Hohlnadel verzichtet werden.

Das vorstehend beschriebene Verfahren hat verschiedene Nachteile. So nimmt dieses Verfahren sehr viel Zeit in Anspruch und bindet des Bedienungspersonal. Weiterhin besteht bei einem zu starken Heraussaugen der Referenzflüssigkeit die Gefahr, dass Luft in Form von Mikrobläschen in die Referenzflüssigkeit gelangt, zumal die Spritze mit dem Spritzenzylinder und dem Spritzenkolben keine ausreichende Dichtigkeit gewährleistet. Darüber hinaus tritt die Referenzflüssigkeit mit vielen neuen Oberflächen innerhalb der Spritze in Kontakt, wodurch sich die Gefahr erhöht, dass die Referenzflüssigkeit verunreinigt wird. Vor allem ist jedoch die Reproduzierbarkeit der Bedingungen beim Eingeben der Referenzflüssigkeit nicht gegeben.

Um diese Nachteile zu überwinden, schlägt die US 4,275,774 eine Vorrichtung zum Eingeben einer Referenzflüssigkeit in ein Messgerät vor, die im wesentlichen einer Spritze ähnelt. Die bekannte Vorrichtung weist einen Hohlraum auf, in den eine geöffnete Ampulle eingesetzt und in dem die derart eingesetzte Ampulle arretiert ist. Die bekannte Vorrichtung weist ferner ein Röhrchen auf, das fest in der Hohlkammer angeordnet ist. Das Röhrchen reicht einerseits in die eingesetzte Ampulle und führt andererseits zu einem Auslass, der letztlich in den Eingabeport des Messgeräts eingeführt wird. Der Hohlraum steht mit einer Druckkammer in Verbindung, wobei der Druck in der Druckkammer mit einem verschiebbaren Kolben erhöht oder gesenkt werden kann. Um nun die Referenzflüssigkeit in das Messgerät zu überführen, wird der Auslass in den Eingabeport eingeführt, um anschließend den Druck in der Druckkammer mit Hilfe des Kolbens zu erhöhen. Dieser Druck wirkt nun auf die Oberfläche der Referenzflüssigkeit innerhalb der Ampulle und treibt die Referenzflüssigkeit durch das Röhrchen in das Messgerät.

Die bekannte Vorrichtung hat den Vorteil, dass es nur einen geringen Lufteinschluss in der eingebrachten Referenzflüssigkeit gibt. Darüber hinaus tritt die Referenzflüssigkeit mit weniger neuen Flächen in Kontakt, so dass die Gefahr einer Verunreinigung der Flüssigkeit geringer ist. Dennoch ist die Anwendung dieser Vorrichtung weiterhin zeitintensiv und die genaue Reproduzierbarkeit des Eingebens der Referenzflüssigkeit in das Messgerät ist nicht gewährleistet.

Neben den oben beschriebenen Verfahren und Vorrichtungen, bei denen der Druck zum Eingeben der Referenzflüssigkeit durch das Bedienungspersonal aufgebracht werden muss, sind Verfahren bekannt, bei denen die Referenzflüssigkeit durch das Messgerät selbst angesaugt wird. So werden beispielsweise Verfahren angewandt, bei denen eine Leitung einerseits in den Eingabeport und andererseits in die Ampulle eingeführt wird, so dass das Messgerät die Referenzflüssigkeit über die Leitung einsaugen kann.

Dieses Verfahren hat den Vorteil, dass die Referenzflüssigkeit wenig Lufteinschlüsse enthält und mit wenigen neuen Flächen in Kontakt kommt, wodurch Verunreinigungen weitgehend vermieden werden. Auch der während des Einsaugens aufgebrachte Druck bzw. Unterdruck ist weitestgehend konstant, so dass die Reproduzierbarkeit der Übertragungsvorgänge verbessert ist. Trotz dieser Verbesserungen ist die Reproduzierbarkeit noch immer nicht optimal.

Ausgehend von dem letztgenannten Stand der Technik besteht die Aufgabe der Erfindung daher darin, eine Vorrichtung zum Einbringen einer Referenzflüssigkeit in ein Messgerät und ein Messgerät zu schaffen, bei der bzw. dem das Einbringen der Referenzflüssigkeit in das Messgerät stets zu annähernd gleichen Bedingungen durchgeführt werden kann. Eine weitere Aufgabe der Erfindung besteht darin, ein Verfahren zum Einbringen einer Referenzflüssigkeit in ein Messgerät zur Ermittlung der Parameter von Flüssigkeitsproben anzugeben, mittels dessen das Einbringen der Referenzflüssigkeit stets zu annähernd gleichen Bedingungen durchgeführt werden kann.

Um Referenzflüssigkeit aus der Ampulle zu entnehmen, kommt häufig auch eine Hohlnadel zum Einsatz, die im Gerät implementiert ist. Die Hohlnadel wird dabei in die geöffnete Ampulle eingeführt, um die Referenzflüssigkeit anschließend in das Gerät zu saugen. Wurde der Ampulle genügend Referenzflüssigkeit entnommen, so kann die Ampulle von der Hohlnadel entfernt werden.

Während des Entnahmevorgangs der Referenzflüssigkeit durch das Gerät muss die Bedienungsperson die Ampulle per Hand an der Hohlnadel halten und positionieren. Durch ständiges Nachführen der Ampulle muss die Bedienungsperson gewährleisten, dass sich die Hohlnadel beim Ansaugen im Flüssigkeitsvolumen befindet, um ein Einsaugen von atmosphärischer Luft zu vermeiden. Für folgende Analysen muss die im Gerät implementierte Hohlnadel sowohl innen als auch außen gereinigt werden, um eventuelle Flüssigkeitsrückstände zu entfernen.

Das vorstehende Verfahren hat verschiedene Nachteile. So nimmt dieses Verfahren sehr viel Zeit in Anspruch und bindet das Bedienungspersonal. Während des Entnehmens der Flüssigkeit positioniert der Bediener die Ampulle an der Hohlnadel. Durch die benutzerindividuelle Eingabe verringert sich die Reproduzierbarkeit der Bedingungen beim Einbringen der Referenzflüssigkeit.

Weiterhin besteht durch die Positionierung per Hand die Gefahr, Luftblasen anstelle der Referenzflüssigkeit in das Gerät einzubringen. Durch die implementierte Hohlnadel ist eine Reinigung nach der Entnahme der Referenzflüssigkeit sowohl innen als auch außen notwendig. Flüssigkeitsrückstände führen bei den nachfolgenden Analysen zu Verfälschungen. Durch die benutzerindividuelle Reinigung verringert sich die Reproduzierbarkeit der folgenden Analysen.

Ein weiteres bekanntes Verfahren besteht in der Entnahme der Referenzflüssigkeit ohne die Unterstützung des Bedieners. Im oder am Gerät befindet sich ein implementiertes Vorratsbehältnis mit einer Vielzahl von mit Referenzflüssigkeit gefüllten Ampullen oder Beuteln. Zur Entnahme der Referenzflüssigkeit aus der Ampulle oder dem Beutel werden diese zerstörend geöffnet. Das Einbringen der Referenzflüssigkeit in das Gerät geschieht vorzugsweise durch Ansaugen der Referenzflüssigkeit und Transport durch ein fluidisches Transportsystem. Der Entnahmevorgang der Referenzflüssigkeit wird durch das Gerät automatisiert gesteuert.

Es ergeben sich bei dem zuvor beschriebenen Verfahren unterschiedliche Nachteile. Durch das implementierte Vorratsbehältnis ist eine individuelle Vorbereitung der Vermessung der Referenzflüssigkeit nicht zu gewährleisten. Solche individuellen Vorbereitungen ergeben sich aus ggf. unterschiedlichen Lagerbedingungen. Einflussparameter sind Temperatur und Equilibrierungszustand der Referenzflüssigkeit mit umgebender Gasatmosphäre.

Das fest im Gerät installierte Fluidsystem erfordert nach jeder Entnahme der Referenzflüssigkeit eine Reinigung der flüssigkeitsführenden Kanäle. Verbleibende Flüssigkeitsrückstände führen bei folgenden Analysen zu Verfälschungen der Messwerte.

Ein weiterer Nachteil ergibt sich ebenfalls aus dem fest im Gerät installierten Fluidsystem. Durch die feste Positionierung der Ampullen oder Beutel ergeben sich sehr lange Transportwege des Fluidsystems. Dabei kann es zu Verfälschungen der Partialdruck-Messwerte durch atmosphärische Luft kommen. Darüber hinaus tritt die Referenzflüssigkeit mit vielen neuen Oberflächen innerhalb des Fluidsystems in Kontakt, wodurch sich die Gefahr erhöht, dass die Referenzflüssigkeit verunreinigt wird.

Die Lösung dieser Aufgabe erfolgt erfindungsgemäß mit den Merkmalen der Patentansprüche 1, 9 und 13. Vorteilhafte Ausführungsformen der Erfindung sind Gegenstand der Unteransprüche.

Die erfindungsgemäße Vorrichtung dient dem Einbringen einer Referenzflüssigkeit in ein Messgerät, mit dessen Hilfe die Parameter von Flüssigkeitsproben ermittelt werden können. Die Vorrichtung umfasst ein separates Leitungselement. Das Leitungselement kann beispielsweise ein Röhrchen sein, das starr ausgebildet ist und aus Glas, Kunststoff oder Metall besteht. Das Leitungselement kann einerseits in einen Eingabeport an dem Messgerät und andererseits in eine Ampulle, die die Referenzflüssigkeit enthält, eingeführt werden. Erfindungsgemäß umfasst die Vorrichtung ferner eine Halteeinrichtung zum Halten der die Referenzflüssigkeit enthaltenden Ampulle, wobei die Halteeinrichtung an dem Messgerät befestigt werden kann. Die Befestigung kann beispielsweise derart erfolgen, dass an der Halteeinrichtung und an dem Messgerät korrespondierende Befestigungsmittel vorgesehen sind.

Allerdings ist es ebenso möglich, nur die Halteeinrichtung mit einem Befestigungsmittel zu versehen, das fast überall am Messgerät anbringbar ist, wie beispielsweise ein Saugnapf oder dergleichen. Das Messgerät ist dabei derart ausgebildet, dass es die Referenzflüssigkeit aus der Ampulle saugen kann.

Um die Referenzflüssigkeit einzubringen, wird zunächst das Leitungselement in den Eingabeport eingeführt, um das freie Ende anschließend in die geöffnete Ampulle einzuführen. Die Ampulle kann im Anschluss daran in die an dem Messgerät zuvor befestigte Halteeinrichtung eingebracht werden. Danach kann die Messeinrichtung die Referenzflüssigkeit durch das Leitungselement aus der Ampulle saugen.

Die erfindungsgemäße Vorrichtung hat neben den Vorteilen, die bereits durch die aus dem Stand der Technik bekannten Vorrichtungen erzielt werden, den weiteren Vorteil, dass eine optimale Reproduzierbarkeit des Einbringvorganges gegeben ist. Dies bedeutet, dass selbst bei aufeinander folgenden Messungen an Referenzflüssigkeiten aus verschiedenen Ampullen, annähernd die gleichen Bedingungen gegeben sind, da jede weitere Ampulle lediglich wieder an der Halteeinrichtung befestigt werden muss, in der sie die gleiche Ausrichtung zu dem Eingabeport hat wie die vorangehende Ampulle. Darüber hinaus ist die Handhabung einfacher und weniger zeitintensiv, da das Bedienungspersonal die Ampulle nicht mehr halten muss, sondern in der Halteeinrichtung befestigen kann.

In einer besonders bevorzugten Ausführungsform der erfindungsgemäßen Vorrichtung weist die Halteeinrichtung eine Vertiefung auf, in die die Ampulle eingebracht werden kann. In eine solche Vertiefung kann die Ampulle beispielsweise eingelegt oder eingestellt werden, ohne dass es weiterer Befestigungsmittel bedürfte. Dies gewährleistet ein schnelles Einbringen und Entnehmen von gefüllten bzw. leeren Ampullen, so dass eine weitere Zeitersparnis erzielt werden kann.

Um eine besonders hohe Reproduzierbarkeit des Einbringvorgangs der Referenzflüssigkeit zu erzielen, ist die Form der Vertiefung derart ausgebildet, dass eingebrachte Ampullen gleicher Form und Größe stets dieselbe räumliche Anordnung haben. Vorzugsweise ist die Form der Vertiefung zu diesem Zweck derart ausgebildet, dass diese der Form zumindest eines Teiles der Ampulle entspricht. Weiter bevorzugt entspricht die Form der Vertiefung einem Teil eines Zylinders, zumal die meisten Ampullen einen zylindrischen unteren Abschnitt aufweisen. Durch die vorgenannten Ausführungsformen ist eine exakte Reproduzierbarkeit der Positionierung der Ampulle in der Vertiefung gegeben, wodurch die Reproduzierbarkeit des Einbringvorganges der Referenzflüssigkeit gewährleistet ist.

In einer weiteren bevorzugten Ausführungsform der Erfindung ist die Halteeinrichtung derart ausgebildet ist, dass die gehaltene Ampulle schräggestellt ist.

Um eine besonders einfache Handhabung zu erzielen, weist die Halteeinrichtung in einer bevorzugten Ausführungsform der Erfindung einen ersten und zweiten Teil auf, die gelenkig miteinander verbunden sind und zusammengeklappt werden können. Bei einer derartigen Ausführung kann die Halteeinrichtung beispielsweise aus einem einstückigen Spritzgussteil bestehen, bei dem die beiden Teile über einen biegbaren Abschnitt miteinander verbunden sind. Die Halteeinrichtung kann durch Zusammenklappen an einem vorspringenden Ansatz des Messgerätes befestigt werden. Dabei umschließen die beiden Teile den vorspringenden Ansatz.

Um eine Beschädigung des Eingabeports zu verhindern, weist das Leitungselement in einer weiteren bevorzugten Ausführungsform der Erfindung an seinem in den Eingabeport einführbaren Ende abgerundete Kanten auf.

Das erfindungsgemäße Messgerät zur Ermittlung der Parameter von Flüssigkeitsproben weist eine Halteeinrichtung zum Halten einer Ampulle auf, in der eine Referenzflüssigkeit enthalten ist. Die Halteeinrichtung ist im Bereich eines Eingabeports an dem Messgerät angeordnet, so dass ein separates Leitungselement, wie beispielsweise ein Röhrchen, einerseits in den Eingabeport und andererseits in die geöffnete Ampulle eingeführt werden kann.

Um dem Bedienungspersonal die Handhabung zu erleichtern, ist die Halteeinrichtung in einer vorteilhaften Ausführungsform des erfindungsgemäßen Messgerätes einstückig mit dem Messgerät ausgebildet, so dass ein vorheriges Anbringen bzw. Befestigen der Halteeinrichtung an dem Messgerät nicht notwendig ist.

In einer besonders bevorzugten Ausführungsform der Erfindung ist die Halteeinrichtung einstückig mit einem Verschlussteil für den Eingabeport ausgebildet. Da die Halteeinrichtung in das Verschlussteil integriert ist, wird die Teilevielfalt reduziert und somit die Handhabung erleichtert. Des weiteren ist das Verschlussteil und somit die Halteeinrichtung in unmittelbarer Nähe des Eingabeports angeordnet, so dass der Übertragungsweg für die Referenzflüssigkeit kurz ausgebildet sein kann. Die Gefahr von Verunreinigungen wird dadurch weiter herabgesetzt.

Die Halteeinrichtung kann ein Bestandteil des Geräts sein. Es kann von diesem leicht abnehmbar und reinigbar sein. Sie kann als "limited use" Artikel für eine zeitlich begrenzte Einsatzdauer ausgelegt sein. Sie kann auch Bestandteil eines Einmalartikels, insbesondere der Messkassette, sein.

Vorzugsweise weist das Messgerät eine die Strömungskanäle und die Sensoren für die Flüssigkeitsproben enthaltende Messkassette auf, die von dem Messgerät entfernt werden kann. An der Messkassette ist vorzugsweise sowohl der Eingabeport als auch die Halteeinrichtung vorgesehen. Die Messkassette kann nach mehrmaligem Messen einfach entsorgt werden, um durch eine neue Messkassette ausgetauscht zu werden. Ein solches Messgerät mit Einwegkassette eignet sich somit sehr gut für die Anwendung in kleineren Arztpraxen o.ä., bei denen eine regelmäßige und aufwendige Wartung des Messgerätes unverhältnismäßig wäre. Die Vorrichtung zum Einbringen der Referenzflüssigkeit kann aber auch an dem eigentlichen Messgerät vorgesehen sein.

Das erfindungsgemäße Verfahren zum Einbringen einer Referenzflüssigkeit in ein Messgerät zur Ermittlung der Parameter von Flüssigkeitsproben weist die folgenden Verfahrensschritte auf. Zunächst wird ein Leitungselement in den Eingabeport des Messgerätes eingeführt. Anschließend wird das Leitungselement in die geöffnete Ampulle, die die Referenzflüssigkeit enthält, eingeführt. Danach wird die Ampulle in eine an dem Messgerät angeordnete Halteeinrichtung eingebracht. Die Halteeinrichtung ist vorzugsweise wie eine der vorbeschriebenen Halteeinrichtungen ausgebildet. Die geschilderten Verfahrensschritte können in beliebiger Reihenfolge durchgeführt werden. Anschließend erfolgt das Einsaugen der Referenzflüssigkeit durch das Messgerät. Bezüglich der Vorteile des erfindungsgemäßen Verfahrens wird auf die vorstehende Beschreibung der Vorrichtung sowie des Messgerätes verwiesen.

Im Folgenden wird die Erfindung an Hand einer beispielhaften Ausführungsform unter Bezugnahme auf die beigefügten Figuren eingehender erläutert.

Es zeigen:
- Fig. 1: eine perspektivische Darstellung der Halteeinrichtung der erfindungsgemäßen Vorrichtung im aufgeklappten Zustand,
- Fig. 2: eine perspektivische Darstellung der Halteeinrichtung von Fig. 1 im zusammengeklappten Zustand,
- Fig. 3a: eine Seitenansicht des Leitungselementes der erfindungsgemäßen Vorrichtung,
- Fig. 3b: den Ausschnitt A von Fig. 3a in geschnittener Darstellung und
- Fig. 4: eine Seitenansicht der an einem Messgerät angeordneten erfindungsgemäßen Vorrichtung in geschnittener Darstellung mit der Halteeinrichtung und dem Leitungselement der Fig. 1 bis 3b.

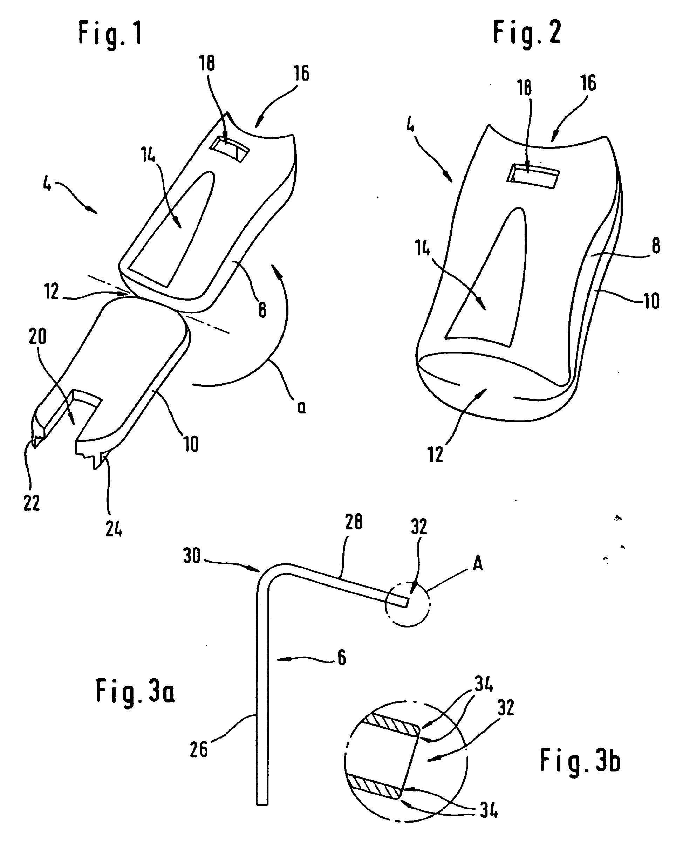

Die erfindungsgemäße Vorrichtung 2 zum Einbringen einer Referenzflüssigkeit in ein Messgerät umfasst eine Halteeinrichtung 4 (Fig. 1 und 2) und ein Leitungselement 6 (Fig. 3a und 3b).

Fig. 1 zeigt die Halteeinrichtung 4 im aufgeklappten Zustand. Die Halteeinrichtung 4 weist einen ersten Teil 8 und einen zweiten Teil 10 auf, die über ein Gelenk 12 gelenkig miteinander verbunden sind. In der dargestellten Ausführungsform ist die Halteeinrichtung 4 einstückig als Kunststoffteil ausgebildet, wobei das Gelenk 12 von einem dünnen und biegsamen Abschnitt zwischen den beiden Teilen 8,10 gebildet wird, so dass ein Zusammenklappen der beiden Teile 8, 10 möglich ist, wie dies an Hand des Pfeils a in Fig. 1 veranschaulicht wird.

In dem ersten Teil 8 ist eine Vertiefung 14 vorgesehen, die im zusammengeklappten Zustand der Halteeinrichtung 4 nach außen zugänglich weist. In diese Vertiefung 14 kann eine nicht näher dargestellte Ampulle, die die Referenzflüssigkeit enthält, eingebracht werden. Die Vertiefung 14 ist dabei derart ausgebildet, dass eingebrachte Ampullen gleicher Form stets dieselbe räumliche Anordnung haben. In der vorliegenden Ausführungsform wird dies dadurch erzielt, dass die Form der Vertiefung 14 der Form zumindest eines Teiles der Ampulle entspricht. Da gängige Ampullen stets einen zylindrischen unteren Abschnitt aufweisen, entspricht die Form der Vertiefung 14 einem Teil eines Zylinders. Die Vertiefung kann auch mit Mitteln zum Verklemmen der Ampulle versehen sein.

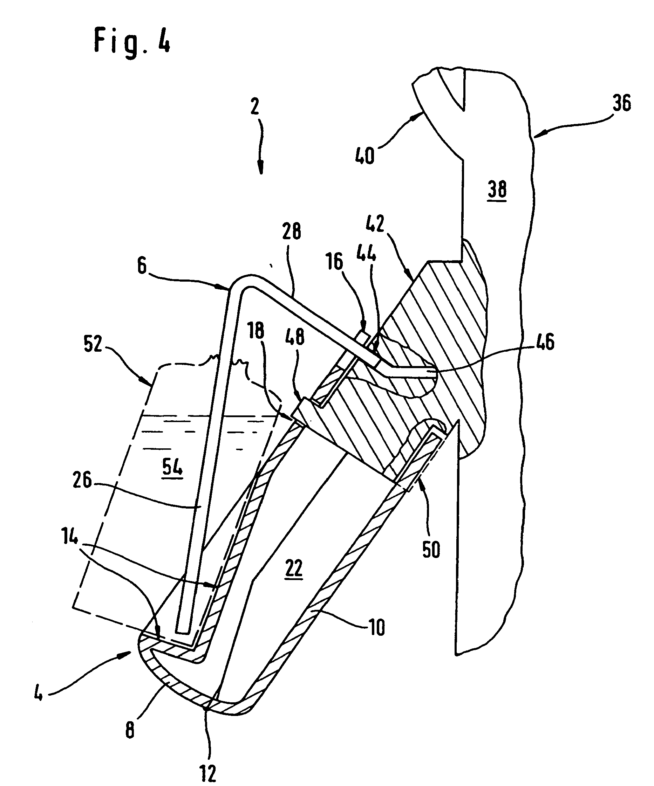

An dem Gelenk 12 abgewandten Ende des ersten Teiles 8 ist ein Einschnitt 16 vorgesehen, der den für das Einführen des Leitungselementes 6 in das Messgerät notwendigen Platz bietet, wie dies später unter Bezugnahme auf Fig. 4 eingehender erläutert wird. Ferner ist in dem ersten Teil zwischen der Vertiefung 14 und dem Einschnitt 16 eine Öffnung 18 angeordnet, durch die ein vorspringender Ansatz an dem Messgerät hindurchgeführt werden kann, wodurch die Halteeinrichtung sicher befestigt ist, wobei auch dies eingehender unter Bezugnahme auf Fig. 4 beschrieben wird.

Der zweite Teil 10 der Halteeinrichtung 4 weist an seinem dem Gelenk 12 abgewandten Ende ebenfalls einen Einschnitt 20 auf. Des weiteren sind an dem zweiten Teil 10 zwei parallel verlaufende und nach innen versetzte Seitenwände 22, 24 vorgesehen, die im zusammengeklappten Zustand (Fig. 2), d. h. nach dem Verschwenken um das Gelenk 12 in Richtung des Pfeils a, innerhalb des ersten Teiles 8 einliegen, der schalenförmig ausgebildet ist.

Das in Fig. 3a dargestellte Leitungselement 6 ist als starres Glas-, Kunststoff- oder auch Metallröhrchen ausgebildet, das einen ersten Schenkel 26 und einen zweiten Schenkel 28 aufweist, die über einen gebogenen Abschnitt 30 miteinander verbunden sind. Der erste Schenkel 26, der später dazu dienen soll, tief in die geöffnete Ampulle eingeführt zu werden, ist etwas länger ausgebildet als der zweite Schenkel 28, der dazu dient, den Weg zwischen dem Ausgang der Ampulle und einem Eingabeport in dem Messgerät zu überbrücken. Der zweite Schenkel 28 liegt auf der Verlängerung der Achse des Probenports und erstreckt sich bis zum Schnittpunkt der Achse des Probenports mit der Achse der in der Halteeinrichtung fixierten Ampulle, während sich der erste Schenkel 26 des Leitungselements 6 von dem vorgenannten Schnittpunkt bis knapp über den inneren Boden der Ampulle erstreckt.

Das dem gebogenen Abschnitt 30 abgewandte Ende 32 des zweiten Schenkels 28 soll also in einen Eingabeport eingeführt werden. Da dies beschädigungsfrei erfolgen muss, damit der Eingabeport nach dem Entfernen des Leitungselements 6 auch wieder sicher schließt, sind die Kanten 34 am Ende 32 des Schenkels 28 abgerundet, wie dies in Fig. 3a zu erkennen ist. Ferner ermöglicht die Abrundung der Kanten 34 ein einfacheres Einführen des Endes 32. Das Leitungselement 6 ist als separates Einzelteil ausgebildet, das nach einmaliger Verwendung bereits entsorgt werden kann, um gegen ein neues Leitungselement ausgetauscht zu werden. Auf diese Weise entfällt ein Reinigen oder Spülen bereits benutzter Leitungselemente.

Fig. 4 zeigt einen Teil des Messgerät 36 zusammen mit der Halteeinrichtung 4. Das Messgerät 36 weist eine Messkassette 38 auf, in der sich sowohl die Strömungskanäle (nicht dargestellt) als auch die Sensoren (nicht dargestellt) für die Flüssigkeitsproben befinden. Die Messkassette 38 ist in das Messgerät 36 eingeschoben und kann mittels des angedeuteten Griffes 40 wieder von dem Messgerät 36 entfernt und entsorgt werden. Bei dieser Ausführungsform ist die Halteeinrichtung an der Messkassette vorgesehen. Grundsätzlich kann die Halteeinrichtung aber auch Bestandteil des eigentlichen Messgeräts sein.

An der Messkassette 38 befindet sich ein von außen zugänglicher, vorspringender Verbindungsansatz 42, an dem in der vorliegenden Ausführungsform ein Eingabeport 44 zum Einbringen einer Referenzflüssigkeit angeordnet ist. An den Eingabeport 44 schließt sich eine Zuführleitung 46 an, die zu den Strömungskanälen und den Sensoren innerhalb der Messkassette 38 führt. Der nach unten geneigte Verbindungsansatz 42 weist an seiner Ober- und Unterseite jeweils einen weiteren vorspringenden Ansatz 48, 50 (letzterer ist gestrichelt dargestellt) auf.

Im Folgenden soll die Vorgehensweise beim Einbringen der Referenzflüssigkeit in das Messgerät 36 beschrieben werden. Zunächst werden die beiden Teile 8, 10 der Halteeinrichtung 4 (Fig. 1) in Richtung des Pfeils a um das Gelenk 12 zusammengeklappt, wodurch der vorspringende Verbindungsansatz 42 an dem Messgerät 36 von den Enden der Teile 8, 10 umschlossen werden soll, die dem Gelenk 12 abgewandt sind. Bei richtiger Ausrichtung bzw. Anordnung der Halteeinrichtung 4 erstreckt sich der Ansatz 48 auf der Oberseite des Verbindungsansatzes 42 in oder durch die Öffnung 18 in dem Teil 8 und der Ansatz 50 auf der Unterseite des Verbindungsansatzes 42 in oder durch den Einschnitt 20 in dem Teil 10, wenn die beiden Teile 8, 10 zusammengeklappt sind. Des weiteren wird der Verbindungsansatz 42 seitlich von den nach innen versetzten Seitenwänden 22, 24 in dem Teil 10 umgeben.

Die Öffnung 18 und der Ansatz 48 sind derart aufeinander abgestimmt, dass nur ein geringes Spiel gegeben ist, wenn der Ansatz 48 in die Öffnung 18 hineinragt, so dass eine sichere gleichbleibende Anordnung zwischen Halteeinrichtung 4 und dem Verbindungsansatz 42 besteht. Ebenso verhält es sich mit dem Ansatz 50 und dem Einschnitt 20. Auch die beiden Seitenwände 22, 24 liegen relativ dicht an dem Verbindungsansatz 42 an.

An den beiden Teilen 8, 10 der Halteeinrichtung können noch Mittel vorgesehen sein, die ein Verbleiben der beiden Teile 8, 10 in dieser zusammengeklappten Position gewährleisten, wie beispielsweise Einrasthaken, jedoch wurde auf deren Darstellung aus Übersichtlichkeitsgründen verzichtet. Des weiteren könnten auch an dem Ansatz 48 Mittel vorgesehen sein, die den Rand der Öffnung 18 nach dem Zusammenklappen der Teile 8, 10 hintergreifen, so dass eine noch sicherere Befestigung erzielt wird. Entsprechendes gilt für den Ansatz 50, der in Fig. 4 gestrichelt dargestellt ist.

Nachdem die Halteeinrichtung 4 an dem Messgerät 36 bzw. der Messkassette 38 befestigt ist, wird das Leitungselement 6 mit dem Ende 32 des zweiten Schenkels 28 in den Eingabeport 44 eingeführt, wobei der erste Schenkel 26 des Leitungselementes 6 anschließend in eine geöffnete Ampulle 52 eingeführt wird, die in Fig. 4 gestrichelt dargestellt ist. Der zweite Schenkel 28 erstreckt sich durch den Einschnitt 16 in dem Teil 8 der Halteeinrichtung 4. Der Einschnitt 16 gewährleistet somit, dass die Halteeinrichtung 4 möglichst nah an dem Eingabeport 44 angeordnet werden kann, ohne diesen zu verdecken. Der somit erzielte kürzere Übertragungsweg zwischen der Ampulle 52 und dem Eingabeport 44 ermöglicht ein kürzeres Leitungselement.

Danach wird die Ampulle 52, deren unterer Abschnitt zylinderförmig ausgebildet ist, in die Vertiefung 14 in der Halteeinrichtung 4 eingebracht. Ein weiteres Halten der Ampulle 52 durch das Bedienungspersonal ist von nun an nicht mehr erforderlich. Abschließend kann das Messgerät die Referenzflüssigkeit 54 durch den ersten Schenkel 26, den gebogenen Abschnitt 30 und den zweiten Schenkel 28 über den Eingabeport 42 in die Zuführleitung 46 saugen, um die Messung an der eingesaugten Referenzflüssigkeit 54 vorzunehmen.

Der besondere Vorteil der erfindungsgemäßen Vorrichtung liegt in der genauen Reproduzierbarkeit von aufeinander folgenden Einsaugvorgängen, da eine Ampulle stets in derselben Ausrichtung angeordnet werden kann, wie eine Ampulle aus einer vorangegangenen Messung. Eine hohe Reproduzierbarkeit der Einbringvorgänge ist somit gewährleistet, was letztlich zu einer hohen Genauigkeit beim Kalibrieren des Messgerätes führt.

## Patentansprüche

1. Vorrichtung zum Einbringen einer Referenzflüssigkeit (54) in ein Messgerät (36) zur Ermittlung der Parameter von Flüssigkeitsproben mit einem separaten Leitungselement (6), das einerseits in einen Eingabeport (44) an dem Messgerät (36) und andererseits in eine Ampulle (52), die die Referenzflüssigkeit (54) enthält, einführbar ist,
wobei die Vorrichtung eine an dem Messgerät (36) befestigbare Halteeinrichtung (4) zum Halten der die Referenzflüssigkeit (54) enthaltenden Ampulle (52) aufweist.

2. Vorrichtung nach Anspruch 1, **dadurch gekennzeichnet, dass** die Halteeinrichtung (4) eine Vertiefung (14) aufweist, in die die Ampulle (52) einbringbar ist.

3. Vorrichtung nach Anspruch 2, **dadurch gekennzeichnet, dass** die Form der Vertiefung (14) derart ausgebildet ist, dass eingebrachte Ampullen (52) gleicher Form stets dieselbe räumliche Anordnung haben.

4. Vorrichtung nach Anspruch 3, **dadurch gekennzeichnet, dass** die Form der Vertiefung (14) der Form zumindest eines Teiles der Ampulle (52) entspricht.

5. Vorrichtung nach Anspruch 4, **dadurch gekennzeichnet, dass** die Form der Vertiefung (14) einem Teil eines Zylinders entspricht.

6. Vorrichtung nach einem der vorangehenden Ansprüche, **dadurch gekennzeichnet, dass** die Halteeinrichtung (4) derart ausgebildet ist, dass die gehaltene Ampulle (52) schräggestellt ist.

7. Vorrichtung nach einem der vorangehenden Ansprüche, **dadurch gekennzeichnet, dass** die Halteeinrichtung (4) einen ersten und zweiten Teil (8, 10) aufweist, die gelenkig miteinander verbunden und zusammenklappbar sind, wobei die Halteeinrichtung (4) durch Zusammenklappen an einem vorspringenden Ansatz (42) des Messgerätes (36) befestigbar ist.

8. Vorrichtung nach einem der vorangehenden Ansprüche, **dadurch gekennzeichnet, dass** das Leitungselement (6) an seinem in den Eingabeport (44) einführbaren Ende (32) abgerundete Kanten (34) aufweist.

9. Messgerät zur Ermittlung der Parameter von Flüssigkeitsproben mit einer Vorrichtung gemäß Anspruch 1, wobei die Halteeinrichtung (4) im Bereich eines Eingabeports (44) an dem Messgerät (36) angeordnet ist, so dass ein separates Leitungselement (6) einerseits in den Eingabeport (44) und andererseits in die geöffnete Ampulle (52) einführbar ist.

10. Messgerät nach Anspruch 9, **dadurch gekennzeichnet, dass** die Halteeinrichtung (4) einstückig mit dem Messgerät (36) ausgebildet ist.

11. Messgerät nach Anspruch 9 oder 10, **dadurch gekennzeichnet, dass** die Halteeinrichtung einstückig mit einem Verschlussteil für den Eingabeport (44) ausgebildet ist.

12. Messgerät nach einem der Ansprüche 9 bis 11, **dadurch gekennzeichnet, dass** das Messgerät (36) eine die Strömungskanäle und die Sensoren für die Flüssigkeitsproben enthaltende und von dem Messgerät (36) entfernbare Messkassette (38) aufweist, an der der Eingabeport (44) vorgesehen und die Halteeinrichtung (4) angeordnet ist.

13. Verfahren zum Einbringen einer Referenzflüssigkeit in ein Messgerät zur Ermittlung der Parameter von Flüssigkeitsproben mit den Verfahrensschritten
Einführen eines Leitungselementes in den Eingabeport des Messgerätes,
Einführen des Leitungselementes in die geöffnete Ampulle, die die Referenzflüssigkeit enthält, und
Einbringen der Ampulle in eine an dem Messgerät angeordnete Halteeinrichtung zum Halten der Ampulle.

## Claims

1. An apparatus for introducing a reference liquid (54) into a measuring instrument (36) for determining the parameters of liquid samples with a separate line element (6), which on the one hand can be introduced into an input port (44) on the measuring instrument (36) and on the other hand into an ampoule (52) which contains the reference liquid (54),
whereby the apparatus has a holding arrangement (4) for holding the ampoule (52) containing the reference liquid (54), said holding arrangement being able to be fixed to the measuring instrument (36).

2. The apparatus according to claim 1, **characterised in that** the holding arrangement comprises a recess (14) into which the ampoule (52) can be introduced.

3. The apparatus according to claim 2, **characterised in that** the shape of the recess (14) is configured in such a way that introduced ampoules (52) of identical shape always have the same spatial arrangement.

4. The apparatus according to claim 3, **characterised in that** the shape of the recess (14) corresponds to the shape of at least a part of the ampoule (52).

5. The apparatus according to claim 4, **characterised in that** the shape of the recess (14) corresponds to a part of a cylinder.

6. The apparatus according to any one of the preceding claims, **characterised in that** the holding arrangement (4) is configured in such a way that the held ampoule (52) is inclined.

7. The apparatus according to any one of the preceding claims, **characterised in that** the holding arrangement (4) comprises a first and second part (8, 10), which are connected to one another in an articulated manner and can be folded together, the holding arrangement (4) being able to be fixed by being folded together to a projecting shoulder (42) of the measuring instrument (36).

8. The apparatus according to any one of the preceding claims, **characterised in that** the line element (6) has rounded edges (34) at its end (32) introducible into the input port (44).

9. A measuring instrument for determining the parameters of liquid samples with an apparatus according to claim 1, whereby the holding arrangement (4) is disposed in the region of an input port (44) on the measuring instrument (36), so that a separate line element (6) can be introduced on the one hand into the input port (44) and on the other hand into the opened ampoule (52).

10. The measuring instrument according to claim 9, **characterised in that** the holding arrangement (4) is designed in one piece with the measuring instrument (36).

11. The measuring instrument according to claim 9 or 10, **characterised in that** the holding arrangement is designed in one piece with a closure part for the input port (44).

12. The measuring instrument according to any one of claims 9 to 11, **characterised in that** the measuring instrument (36) comprises a measurement cassette (38) which contains the flow channels and the sensors for the liquid samples and is removable from the measuring instrument (36), the input port (44) being provided on and the holding arrangement (4) disposed on said measurement cassette.

13. A method for introducing a reference liquid into a measuring instrument for determining the parameters of liquid samples with the process steps
introduction of a line element into the input port of the measuring instrument,
introduction of the line segment into the opened ampoule which contains the reference liquid, and
introduction of the ampoule into a holding arrangement for holding the ampoule, said holding arrangement being disposed on the measuring instrument.

## Revendications

1. Dispositif de transfert d'un liquide de référence (54) dans un instrument de mesure (36) pour déterminer les paramètres des échantillons de liquide grâce à un élément de conduite (6) séparé qui peut être introduit d'une part dans un port d'entrée (44) situé sur l'instrument de mesure (36) et d'autre part dans une ampoule (52) qui contient le liquide de référence (54), dans lequel le dispositif présente un dispositif de maintien (4) pouvant être fixé sur l'instrument de mesure (36) afin de maintenir l'ampoule (52) contenant le liquide de référence (54).

2. Dispositif selon la revendication 1 **caractérisé en ce que** le dispositif de maintien (4) présente un renfoncement (14) dans lequel l'ampoule (52) peut être mise en place.

3. Dispositif selon la revendication 2 **caractérisé en ce que** la forme du renfoncement (14) est conçue de manière à ce que des ampoules (52) de même forme mises en place ont toujours la même disposition dans l'espace.

4. Dispositif selon la revendication 3 **caractérisé en ce que** la forme du renfoncement (14) correspond à la forme d'au moins une partie de l'ampoule (52).

5. Dispositif selon la revendication 4 **caractérisé en ce que** la forme du renfoncement (14) correspond à une partie d'un cylindre.

6. Dispositif selon l'une quelconque des revendications précédentes **caractérisé en ce que** le dispositif de maintien (4) est conçu de manière à ce que l'ampoule (52) maintenue soit inclinée.

7. Dispositif selon l'une quelconque des revendications précédentes **caractérisé en ce que** le dispositif de maintien (4) présente une première et seconde parties (8, 10) qui sont reliées l'une à l'autre de manière souple et sont pliables, le dispositif de maintien pouvant être fixé par repli sur un élément supérieur en saillie (42) de l'instrument de mesure (36).

8. Dispositif selon l'une quelconque des revendications précédentes **caractérisé en ce que** l'élément de conduite (6) présente des bords arrondis (34) à son extrémité (32) pouvant être introduite dans le port d'entrée (44).

9. Instrument de mesure pour déterminer les paramètres des échantillons de liquide grâce à un dispositif selon la revendication 1, le dispositif de maintien (4) étant disposé dans la zone d'un port d'entrée (44) situé sur l'instrument de mesure (36), de sorte qu'un élément de conduite (6) séparé peut être introduit d'une part dans le port d'entrée (44) et d'autre part dans une ampoule (52) ouverte.

10. Dispositif selon la revendication 9 **caractérisé en ce que** le dispositif de maintien (4) est conçu d'un seul tenant avec l'instrument de mesure (36).

11. Instrument de mesure selon la revendication 9 ou 10 **caractérisé en ce que** le dispositif de maintien est conçu d'un seul tenant avec un élément d'obturation pour le port d'entrée (44).

12. Instrument de mesure selon l'une quelconque des revendications 9 à 11 **caractérisé en ce que** l'instrument de mesure (36) présente un caisson de mesure (38) contenant les canaux de flux et les capteurs pour les échantillons de liquide et pouvant être éloigné de l'instrument de mesure (36), sur lequel le port d'entrée (44) est prévu et le dispositif de maintien (4) est disposé.

13. Procédé de transfert d'un liquide de référence dans un instrument de mesure pour déterminer les paramètres des échantillons de liquide avec les étapes de procédé suivantes :
■ introduction d'un élément de conduite dans le port d'entrée de l'instrument de mesure,
■ introduction d'un élément de conduite dans l'ampoule ouverte qui contient le liquide de référence et
■ transfert de l'ampoule dans un dispositif de maintien disposé sur l'instrument de mesure pour maintenir l'ampoule.
